# EUROPEAN PATENT SPECIFICATION

(11) **EP 4 367 119 B1**
(45) Date of publication and mention of the grant of the patent: **21.10.2026**
(21) Application number: 22747323.8
(22) Date of filing: 08.07.2022
(51) Int. Cl.: C07D 471/08, C07F 13/00, C07F 15/00, C08F 283/01, C08K 5/00, C09D 167/00

(54) **BISPIDONE LIGANDS AND TRANSITION METAL COMPLEXES THEREOF**
BISPIDON-LIGANDEN UND ÜBERGANGSMETALLKOMPLEXE DAVON
LIGANDS DE BISPIDONE ET COMPLEXES DE MÉTAL DE TRANSITION ASSOCIÉS

(30) Priority: 09.07.2021 EP 21184778; 24.08.2021 EP 21192796
(43) Date of publication of application: 15.05.2024
(73) Proprietor: Umicore, 1000 Brussels (BE)
(72) Inventor: VERCAEMST, Carl, 8000 Brugge (BE); DE VREESE, Rob, 8000 Brugge (BE)
(86) International application number: PCT/EP2022/069043
(87) International publication number: WO 2023/281046

(56) References cited:
- WO-A1-2008/003652
- WO-A1-2011/124282
- WO-A1-2012/093250
- WO-A1-2013/083632
- WO-A1-2015/011430
- WO-A1-2017/085154
- WO-A1-2018/015333
- WO-A1-2020/008203
- WO-A1-2020/008205
- US-A1- 2014 114 073

## Description

### TECHNICAL FIELD

The present invention relates to novel bispidone ligands and transition metal complexes thereof, especially iron and manganese complexes thereof. Furthermore, the present invention also relates to the use of said bispidone ligands and complexes thereof as a siccative agent in curable liquid compositions and as curing catalyst in unsaturated polyester resins.

### INTRODUCTION

Bispidone ligands are bicyclic diamine compounds which are widely known for their use as chelating agents, and have found applications in catalysis, pharmaceuticals and polymer chemistry. Recently, iron and manganese complexes comprising bispidone ligands were found effective as drying agents in paint formulations.

Specifically, WO 2008/003652 relates to curing agents for air-drying alkyd-based resins, coatings, such as paint, varnish or wood stain, inks and linoleum floor coverings, based on an iron/manganese complex containing tetradentate, pentadentate or hexadentate nitrogen donor ligands. US2014/114073A1 relates to 3,7-Diazabicyclo[3.3.1]nonane compounds as ligands for transition metal complexes.

More recently, WO 2020/008205 reported new bispidone ligands comprising heteroaryl groups other than 2-pyridyl, which are directly attached to the bicyclic moiety within bispidones, were detected to catalyse faster curing of oxidatively curable coating formulations than would have been expected given their close structural similarity with analogous complexes comprising bis(2-pyridyl)bispidones. In parallel, improved catalytic activity was also observed for these ligands in the curing of unsaturated resins as reported in WO 2020/008203.

Although good drying ability, the present inventors found that the drying characteristics of bispidone complexes according to the prior art suffer from severe loss of drying capability upon storage. It is thus an object of the present invention to provide new bispidone ligands and complexes thereof, which allow for improved stability of drying capacity after storage up to at least 2 months, preferably at least 3 months or even more.

### SUMMARY

The current invention provides in a solution for at least one of the above mentioned problems by providing novel bispidone ligands, as described in claim 1, and transition metal complexes thereof, as well as curable liquid compositions thereof. The inventors have found that, alkyd resin compositions comprising a mixture of transition metal ions and bispidone ligands according to the invention do not suffer a significant performance loss up to a shelf life of about 2 to 3 months or even more.

### DESCRIPTION OF THE FIGURES

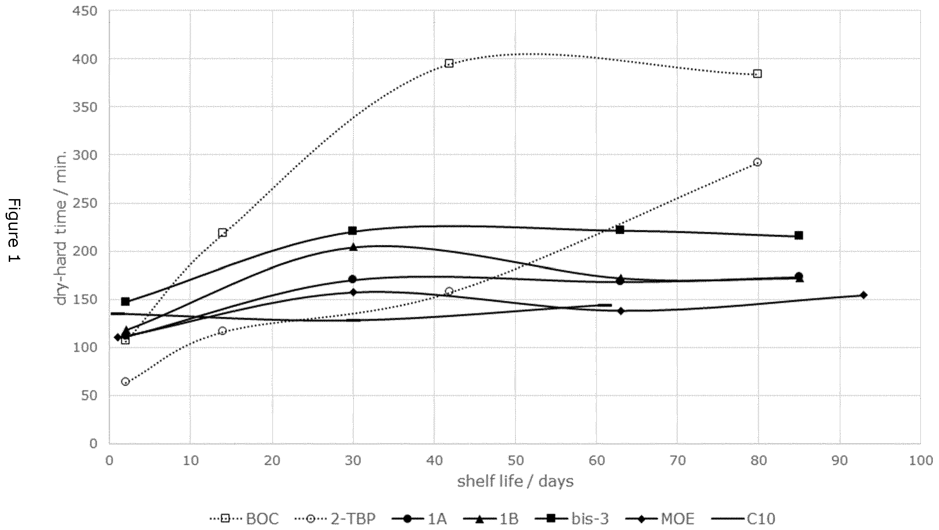

Figure 1 shows the drying performance, expressed in terms of dry-hard time, as a function of the number of days of shelf life of an alkyd resin composition comprising a mixture of bispidone ligands and iron ions.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention. As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints. All percentages are to be understood as percentage by weight, abbreviated as "wt.%" or as volume per cent, abbreviated as "vol.%", unless otherwise defined or unless a different meaning is obvious to the person skilled in the art from its use and in the context wherein it is used.

The term "bispidine" is 3,7-diazabicyclo[3.3.1]nonane and is an organic compound that is classified as a bicyclic diamine. Bispidine and derivatives thereof have use as a chelating agent. In the context of the present invention, the term "bispidine" is to be considered as a compound having a 3,7-diazabicyclo[3.3.1]nonane structure as well as synthetic derivatives of said structure as well as isomers and hydrates thereof. The term "bispidon" is to be considered synonymous to the term "bispidone" and refers to bispidine compounds having a ketone or ketal functionality, preferably a ketone functionality on the C-[9] carbon of the bispidine structure as well as isomers, salts and hydrates thereof.

In a first aspect, the present invention provides a multidentate ligand L_{B} according to formula (I) or (II), wherein: 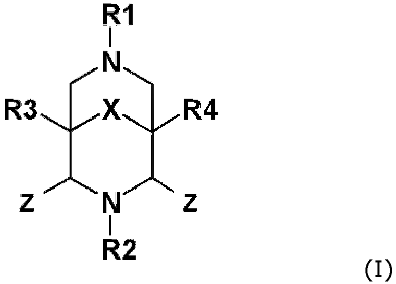
R1 and R2 are independently selected from the group consisting of:
   - a group D containing a heteroatom capable of coordinating to a transition metal;
   - a -C1-C24-alkyl and/or a -C1-C22-substituted-alkyl;
   - a -C6-C10-aryl;
   - a -C1-C4-alkyl-C6-C10-aryl;
R3 and R4 are independently selected from: -(CH₂)ₙ-C(=O)OR5, wherein n is an integer from 0 to 4, preferably n is 0 or 1 and more preferably n is 0, and wherein R5 is selected from the group consisting of -C5-C12-alkyl, -C5-C12-hydroxyalkyl, -C2-C12-alkyl-O-C1-C10-alkyl, -C2-C12-alkyl-O-C2-C12-alkyl-O-C1-C10-alkyl, -C2-C6-alkyl-O-C6-C10-aryl and -C1-C12-alkyl-C6-C10-aryl, preferably R3 and R4 are independently selected from: -(CH₂)ₙ-C(=O)OR5, wherein n is an integer from 0 to 4, preferably n is 0 or 1 and more preferably n is 0, and wherein R5 is selected from the group consisting of -C5-C10-alkyl, -C5-C10-hydroxyalkyl, -C2-C10-alkyl-O-C1-C10-alkyl, and -C1-C10-alkyl-C6-C10-aryl;
X is selected from: -C(=O)-, a ketal derivative of -C(=O)-, a hemiketal derivative of -C(=O)-, a thioketal derivative of -C(=O)-, and -[C(R6)₂]y- wherein y is an integer between 0 and 3; each R6 is independently selected from hydrogen, hydroxyl, -O-C1-C24-alkyl, -O-benzyl, -O-(C=O)-C1-C24-alkyl, and -C1-C24-alkyl;
z groups are same monocyclic or dicyclic heteroaromatic donor groups of the form: 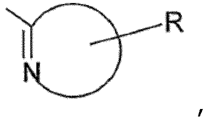 wherein R is H, F, Cl, Br, hydroxyl, C1-C4-alkoxy, -NH-CO-H, - NH-CO-C1-C4-alkyl, -NH₂, or a C1-C4-alkyl; 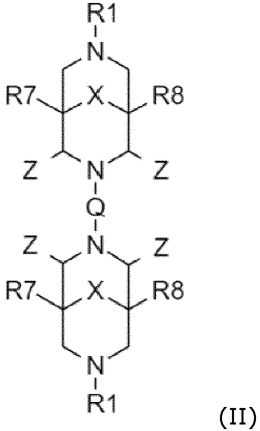
R1 is selected from the group consisting of:
   - a group D containing a heteroatom capable of coordinating to a transition metal;
   - a -C1-C24-alkyl and/or a -C1-C22-substituted-alkyl;
   - a -C6-C10-aryl;
   - a -C1-C4-alkyl-C6-C10-aryl;
R7 and R8 are independently selected from: -(CH₂)ₙ-C(=O)OR9, wherein n is an integer from 0 to 4 and wherein R9 is selected from the group consisting of -C1-C10-alkyl, -C2-C10-hydroxyalkyl, -C2-C10-alkyl-O-C1-C10-alkyl, and -C1-C10-alkyl-C6-C10-aryl;
Q is selected from: a -C5-C24-alkylene and a -C5-C22-substituted-alkylene;
X is selected from: -(C=O)-, a ketal derivative of -(C=O)-, a thioketal derivative of - (C=O)-, and -[C(R6)₂]y- wherein y is an integer between 0 and 3; each R6 is independently selected from hydrogen, hydroxyl, -O-C1-C24-alkyl, -O-benzyl, -O-(C=O)-C1-C24-alkyl, and -C1-C24-alkyl;
z groups are same monocyclic or dicyclic heteroaromatic donor groups of the form: 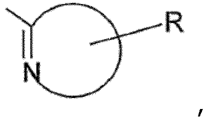 wherein R is H, F, Cl, Br, hydroxyl, -C1-C4-alkoxy, -NH-CO-H, - NH-CO-C1-C4-alkyl, -NH₂, or a -C1-C4-alkyl. Preferably, R is -C0-C4 alkyl.

In a preferred embodiment, the present invention provides a ligand according to the first aspect of the invention, wherein n is 0 or 1, and preferably, wherein n is 0.

In a preferred embodiment, the present invention provides a ligand according to the first aspect of the invention, according to formula (I), wherein R5 is selected from the group consisting of -C5-C8-alkyl, -C2-C8-hydroxyalkyl, -C2-C6-alkyl-O-C1-C6-alkyl, and C2-C3-alkyl-O-C2-C3-alkyl-O-C1-C4-alkyl and -C5-C8-alkyl-C6-C10-aryl. Preferably, R5 is selected from the group consisting of C5-C8-alkyl, and more preferably n is 0 and R5 is -C5-alkyl, -C6-alkyl, -C7-alkyl or -C2-C3-alkyl-O-C1-C4-alkyl.

In a preferred embodiment, the present invention provides a ligand according to the first aspect of the invention, according to formula (II), wherein R9 is selected from the group consisting of -C1-C8-alkyl, -C2-C8-hydroxyalkyl, -C2-C4-alkyl-O-C1-C4-alkyl, and -C1-C8-alkyl-C6-C10-aryl. Preferably, R9 is selected from the group consisting of C1-C4-alkyl, and more preferably n is 0 and R9 is methyl or ethyl.

In a preferred embodiment, the present invention provides a ligand according to the first aspect of the invention, wherein the group D containing a heteroatom capable of coordinating to a transition metal is selected from the group consisting of:
- an optionally substituted tertiary amine of the form -C2-C4-alkyl-NR10R11, in which R10 and R11 are independently selected from the group consisting of straight chain, branched or cyclo C1-C12 alkyl, benzyl, wherein the -C2-C4-alkyl-of the -C2-C4-alkyl-NR10R11 may be substituted by 1 to 4 -C1-C2-alkyl, or may form part of a -C3-C6-alkyl ring, and in which R10 and R11 may together form a saturated ring containing one or more other heteroatoms;
- a heterocycloalkyl selected from the group consisting of: pyrrolinyl, pyrrolidinyl, morpholinyl, piperidinyl, piperazinyl, azepanyl, 1,4-piperazinyl, tetrahydrothiophenyl, tetrahydrofuranyl, tetrahydropyranyl, and oxazolidinyl, wherein the heterocycloalkyl may be connected to the ligand via any atom in the ring of the selected heterocycloalkyl;
- a -C1-C6-alkyl-heterocycloalkyl, wherein the heterocycloalkyl of the -C1-C6-alkyl-heterocycloalkyl is selected from the group consisting of: piperidinyl, 1,4-piperazinyl, tetrahydrothiophenyl, tetrahydrofuranyl, pyrrolidinyl, and tetrahydropyranyl, wherein the heterocycloalkyl may be connected to the -C1-C6-alkyl via any atom in the ring of the selected heterocycloalkyl; and,
- a -C1-C6-alkyl-heteroaryl, wherein the heteroaryl of the -C1-C6-alkyl-heteroaryl is selected from the group consisting of: pyridinyl, pyrimidinyl, pyrazinyl, triazolyl, pyridazinyl, 1,3,5-triazinyl, quinolinyl, isoquinolinyl, quinoxalinyl, imidazolyl, pyrazolyl, benzimaidazolyl, thiazolyl, oxazolidinyl, pyrrolyl, carbazolyl, indolyl, and isoindolyl, wherein the heteroaryl may be connected to the C1-C6-alkyl via any atom in the ring of the selected heteroaryl and the selected heteroaryl is optionally substituted by a group selected from the group consisting of a -C1-C4-alkyl, -C0-C6-alkyl-phenol, -C0-C6-alkyl-thiophenol, -C2-C4-alkyl-thiol, -C2-C4-alkyl-thioether, -C2-C4-alkyl-alcohol, -C2-C4-alkyl-amine, and a -C2-C4-alkyl carboxylate.

In a preferred embodiment, the present invention provides a ligand according to the first aspect of the invention, wherein z groups are independently selected from the group consisting of pyridin-2-yl, thiazol-2-yl, thiazol-4-yl, pyrazin-2-yl, quinolin-2-yl, pyrazol-3-yl, pyrazol-1-yl, pyrrol-2-yl, imidazol-2-yl, imidazol-4-yl, benzimidazol-2-yl, pyrimidin-2-yl, 1,2,4-triazol-3-yl, 1,2,4-triazol-1-yl, 1,2,3-triazol-1-yl, 1,2,3-triazol-2-yl and 1,2,3-triazol-4-yl, each of which may be optionally substituted by one or more groups independently selected from the group consisting of -F, -Cl, -Br, -OH, - O-C1-C4 alkyl, -NH-CO-H, -NH-CO-C1-C4 alkyl, -NH₂, -NH-C1-C4 alkyl, and -C1-C4 alkyl.

In a more preferred embodiment, the present invention provides a ligand according to the first aspect of the invention, wherein z groups are same heteroaromatic groups of the form: pyridine-2-yl, thiazol-2-yl and thiazol-4-yl.

In a preferred embodiment, the present invention provides a ligand according to the first aspect of the invention, according to formula (I) wherein one of R1 and R2 is selected from the group consisting of, or according to formula (II) wherein R1 is selected from the group consisting of: a non-aromatic hydrocarbon group, the non-aromatic hydrocarbon group being a C1-C8 alkyl chain, preferably a C1-C4 alkyl chain and more preferably methyl or ethyl. More preferably, R2 is a non-aromatic hydrocarbon group, the non-aromatic hydrocarbon group being a C1-C8 alkyl chain, preferably a C1-C4 alkyl chain and more preferably methyl or ethyl, and most preferably R2 is methyl.

In a preferred embodiment, according to formula (I), the present invention provides a ligand according to the first aspect of the invention, according to formula (I) wherein one of R1 and R2 is selected from the group consisting of, or according to formula (II) wherein R1 is selected from the group consisting of: an optionally substituted tertiary amine of the form -C2-C4 alkyl-NR10R11, in which R10 and R11 are independently selected from the group consisting of straight chain, branched or cyclo C1-C12 alkyl, -CH₂-C₆H₅, wherein the C₆H₅ is optionally substituted by -C1-C4-alkyl or -O-C1-C4-alkyl, and pyridin-2-ylmethyl wherein the pyridine is optionally substituted by C1-C4-alkyl, the -C2-C4-alkyl- of the -C2-C4-alkyl-NR10R11 may be substituted by 1 to 4 C1-C2-alkyl, or may form part of a C3-C6 alkyl ring, and in which R10 and R11 may together form a saturated ring containing one or more other heteroatoms. Preferably, said optionally substituted tertiary amine is of the form -C2-alkyl-NR10R11 or -C3-alkyl-NR10R11. Preferably, NR10R11 is selected from group consisting of: -NMe₂, -NEt₂, -N(i-Pr)₂.

In a preferred embodiment, the present invention provides a ligand according to the first aspect of the invention, wherein X is -(C=O)-.

In a preferred embodiment, the present invention provides a ligand according to the first aspect of the invention, according to formula (I) wherein one of R1 and R2 is selected from the group consisting of, or according to formula (II) wherein R1 is selected from the group consisting of: Me, -CH₂-C₆H₅, and pyridin-2-ylmethyl, wherein the pyridin-2-ylmethyl is optionally substituted by C1-C4 alkyl. Preferably, one of R1 and R2 is a pyridin-2-ylmethyl, optionally substituted by C1-C4-alkyl, but preferably not substituted.

In a preferred embodiment, the present invention provides a ligand according to the first aspect of the invention, according to formula (I), wherein R1 is pyridin-2-ylmethyl, R2 is methyl, R3 and R4 are -C(=O)-O-C₅H₁₁, X is C=O and z is 2-pyridinyl.

In a preferred embodiment, the present invention provides a ligand according to the first aspect of the invention, according to formula (I), wherein R1 is pyridin-2-ylmethyl, R2 is methyl, R3 and R4 are -C(=O)-O-(1-methyl-2-methoxy-ethyl), X is C=O and z is 2-pyridinyl.

In a preferred embodiment, the present invention provides a ligand according to the first aspect of the invention, according to formula (II), wherein Q is selected from the group consisting of C5-C8-alkylene.

In a preferred embodiment, the present invention provides a ligand according to the first aspect of the invention, according to formula (II), wherein R1 is pyridin-2-ylmethyl, Q is C6-alkylene, R7 and R8 are -C(=O)-OMe, X is C=O and z is 2-pyridinyl.

In a second aspect, the present invention provides a transition metal complex comprising a transition metal and a multidentate ligand L_{B} according to the first aspect of the invention.

In a preferred embodiment, said transition metal is selected from the group comprising: Mn(II), Mn(III), Mn(IV), Mn(V), Cu(I), Cu(II), Cu(III), Fe(II), Fe(III), Fe(IV), Fe(V), Co(I), Co(II), Co(III), Ti(II), Ti(III), Ti(IV), V(II), V(III), V(IV), V(V), Mo(II), Mo(III), Mo(IV), Mo(V), Mo(VI), W(IV), W(V) and W(VI); preferably from the group consisting of Fe(II), Fe(III), Fe(IV), Fe(V), Mn(II), Mn(III), Mn(IV), and Mn(V); and more preferably from the group consisting of Fe(II), Fe(III), Mn(II), Mn(III). Most preferably, said transition metal is selected from the group comprising: Fe(II) and Fe(III).

In a preferred embodiment, the present invention provides a transition metal complex is selected from the group consisting of [FeL_{B}Cl]Cl, [FeL_{B}(H₂O)](PF₆)₂, [FeL_{B}Cl]PF₆, [FeL_{B}(H₂O)](BF₄)₂, [FeL_{B}Cl] carboxylates, [FeL_{B}(H₂O)] carboxylates and bicarboxylates.

In a further aspect, the present invention provides a process for preparing a transition metal complex according to the second aspect of the invention, comprising the step of mixing a multidentate ligand L_{B} according to the first aspect of the invention with a transition metal or a transition metal compound.

In a further aspect, the present invention provides a siccative composition comprising a mixture of a transition metal and a multidentate ligand L_{B} according to the first aspect of the invention. Preferably, said transition metal is comprised in an amount of 0.001 to 1.00 wt.%, relative to the total weight of said siccative composition, more preferably in an amount of 0.01 to 0.10 wt.% and even more preferably in an amount of 0.02, 0.04, 0.06, 0.08 or 0.10 wt.%, or any value there in between.

In a further aspect, the present invention provides a siccative composition comprising a transition metal complex according to the third aspect of the invention. Preferably, said transition metal is comprised in an amount of 0.001 to 1.00 wt.%, relative to the total weight of said siccative composition, more preferably in an amount of 0.01 to 0.10 wt.% and even more preferably in an amount of 0.02, 0.04, 0.06, 0.08 or 0.10 wt.%, or any value there in between.

In a third aspect, the present invention provides a curable liquid composition comprising: a) from 1 to 90 wt.% of an alkyd-based resin or of an unsaturated polyester resin; and, b) from 0.0001 to 1.0 wt.% of a siccative or curing catalyst, respectively, relative to the total weight of said composition, said siccative or curing catalyst consisting essentially of a mixture of a transition metal and a multidentate ligand L_{B} according to the first aspect of the invention, and/or of a transition metal complex according to the second aspect of the invention. Preferably, said transition metal is selected from the group comprising: Mn(II), Mn(III), Mn(IV), Mn(V), Cu(I), Cu(II), Cu(III), Fe(II), Fe(III), Fe(IV), Fe(V), Co(I), Co(II), Co(III), Ti(II), Ti(III), Ti(IV), V(II), V(III), V(IV), V(V), Mo(II), Mo(III), Mo(IV), Mo(V), Mo(VI), W(IV), W(V) and W(VI); preferably from the group consisting of Fe(II), Fe(III), Fe(IV), Fe(V), Mn(II), Mn(III), Mn(IV), and Mn(V); and more preferably from the group consisting of Fe(II), Fe(III), Fe(IV), Fe(V). Most preferably, said transition metal is selected from the group comprising: Fe(II) and Fe(III).

The inventors have found the ligands according to the first aspect of the invention provide improved drying characteristics for coating compositions when mixed with iron and/or manganese ions in situ. Likewise, it was found that iron and/or manganese complexes according to the second aspect of the invention provide excellent drying characteristics for coating compositions. Suitable coating compositions such as the curable liquid compositions according to the third aspect of the invention comprise alkyd-based resins, coatings, inks, and linoleum floor coverings. Preferred ligands contain a tetradentate, pentadentate or hexadentate nitrogen donor ligand. Whilst certain paints/inks contain unsaturated oils/acids as cross-linking agent, most of them contain alkyd-based resins that contain unsaturated groups. The alkyd-based air-drying coatings to which the siccative of the present invention can be added, comprise coatings, such as paint, varnish or wood stain, and also includes inks and linoleum floor coverings and the like. The siccative is equally applicable to setting paints/inks/print which do not contain alkyd-based resins.

The coatings, inks, and linoleum floor coverings may also include compositions wherein besides the alkyd based binder also other binders are present, e.g. compositions comprising 1) an alkyd-based binder and 2) a polyacrylate and/or a polyurethane binder. Conventional air-drying alkyds can be obtained by a polycondensation reaction of one or more polyhydric alcohols, one or more polycarboxylic acids or the corresponding anhydrides, and long chain unsaturated fatty acids or oils.

In a preferred embodiment, the present invention provides a curable liquid composition according to the third aspect of the invention, wherein the content of the siccative is between 0.0001 and 0.5 wt.%, relative to the total weight of said composition, preferably between 0.0001 and 0.1 wt.%.

In a preferred embodiment, the present invention provides a curable liquid composition according to the third aspect of the invention, further comprising between 0.001 and 0.1 wt.% of antioxidants, relative to the total weight of said composition, between 0.002 and 0.05 wt.%, whereby said antioxidant is preferably di-tert-butyl hydroxy toluene, ethoxyquine, alpha-tocopherol, and/or 6-hydroxy-2,5,7, 8-tetramethylchroman-2-carboxylic acid, more preferably alpha-tocopherol.

In a preferred embodiment, the present invention provides a curable liquid composition according to the third aspect of the invention, further containing between 0.001 and 90 wt.%, relative to the total weight of the composition, of a polyhydric alcohol, such as but not limited to ethylene glycol, propylene glycol, diethylene glycol, dipropylene glycol, glycerol, pentaerythritol, dipenta erythritol, neopentyl glycol, trimethylol propane, trimethylol ethane, di-trimethylol propane and/or 1,6-hexane diol.

Preferably, said composition comprises 0.1 to 50 wt.% ethyleneglycol, propylene glycol, or glycerol, more preferably 0.3 to 5 wt.% of ethyleneglycol, propylene glycol or glycerol.

Due to its presence in naturally occurring oils, glycerol is a widely encountered polyol. Other examples of suitable polyhydric alcohols include: pentaerythritol, dipentaerythritol, ethylene glycol, diethylene glycol, propylene glycol, neopentyl glycol, trimethylol propane, trimethylol ethane, di-trimethylol propane and 1,6-hexane diol. Additionally or alternatively, polycarboxylic acids and the corresponding anhydrides, used to synthesize alkyds, may be included, comprising aromatic, aliphatic and cycloaliphatic components. Typical examples of such polyacids include: phthalic acid and its regio-isomeric analogues, trimellitic acid, pyromellitic acid, pimelic acid, adipic acid, azelaic acid, sebacic acid, maleic acid, fumaric acid and tetra-hydrophthalic acid.

Suitable drying fatty acids, semi-drying fatty acids or mixture thereof, useful herein, are ethylenically unsaturated conjugated or non-conjugated C2-C24 carboxylic acids, such as oleic, ricinoleic, linoleic, linolenic, licanic acid and eleostearic acids or mixture thereof, typically used in the form of mixtures of fatty acids derived from natural or synthetic oils. By semi-drying and drying fatty acids is meant fatty acids that have the same fatty acid composition as the oils they are derived from.

Suitable organic solvents to dilute the air-drying alkyds of the invention include aliphatic, cycloaliphatic and aromatic hydrocarbons, alcohol ethers, alcohol esters and N-methylpyrrolidone. However it may also be an aqueous carrier containing the alkyd resin in the form of an emulsion and a suitable emulsifier as is well known in the art.

The composition of the present invention may contain colourants, pigment, anti-corrosive pigment, and/or extender pigment and/or a dye. It may further contain, if necessary, plasticizer, surface-controlling agents, anti-silking agent, an anti-skinning agent, a defoaming agent, a rheological controlling agent and/or an ultraviolet absorber.

The addition of the siccative itself is done with conventional techniques, known to the person skilled in the art. The siccative is either added during the production of the alkyd based resins, coatings, inks, and linoleum floor coverings, or is added under stirring to them before use.

The composition of the present invention is preferably stored under an inert atmosphere, for example nitrogen or carbon dioxide.

In a final aspect, the present invention provides a use of a mixture of a transition metal and a multidentate ligand L_{B} according to the first aspect of the invention; and/or of a transition metal complex according to the third aspect of the invention as a drying agent in a coating formulation.

Synthetic procedures for preparing a multidentate ligand L_{B} according to formula (I) are reported e.g. in US 2013/072685 and US 2014/0114073. Such procedures invariably consist essentially of a transesterification of an alcohol with dimethyl-1,3-acetonedicarboxylate and subsequently formation of the bicyclic bispidone structure. Unfortunately, such procedures are difficult to optimize, especially when a variety of bispidone ligands having a different functionality for the R3 and R4 groups. Therefore, the present invention provides a method for preparing a multidentate ligand L_{B} according to formula (I), comprising the steps of:
i. a first Mannich addition of an alkylamine and an aldehyde to a dialkyl-1,3-acetonedicarboxylate, thereby obtaining a first intermediate;
ii. a second Mannich addition of an alkyl amine and an aldehyde to said first intermediate, thereby obtaining a second intermediate; and subsequently
iii. a transesterification of alkyl groups in said second intermediate using an alcohol.

The ketone functionality in the obtained bispidone ligand L_{B} according to formula (I) may further be functionalised, e.g. to form a ketal. In a preferred embodiment, said first Mannich addition consists of the addition of methylamine and 2-pyridinylaldehyde to a dialkyl-1,3-acetonedicarboxylate. Preferably, said second Mannich addition consists of the addition of 2-(aminomethyl)pyridine and formaldehyde to said first intermediate. Preferably, said dialkyl-1,3-acetonedicarboxylate comprises one or two C1-C4 alkyl groups, preferably two methyl or ethyl groups, and more preferably two methyl groups. Such alkyl derivatives allow for excellent reaction conditions. Preferably, the alcohol used in the transesterification step is represented by the general formula R5-OH, wherein R5 is selected from the group consisting of -C5-C12-alkyl, - C5-C12-hydroxyalkyl, -C2-C12-alkyl-O-C1-C10-alkyl, -C2-C12-alkyl-O-C2-C12-alkyl-O-C1-C10-alkyl, -C2-C6-alkyl-O-C6-C10-aryl and -C1-C12-alkyl-C6-C10-aryl, and preferably wherein R5 is selected from the group consisting of -C5-C10-alkyl, - C5-C10-hydroxyalkyl, -C2-C10-alkyl-O-C1-C10-alkyl, and -C1-C10-alkyl-C6-C10-aryl. Most preferably, the alcohol used in the transesterification step is selected from: HOC₅H₁₁ and HO-(C2-C4-alkyl-O-(C1-C4-alkyl)).

### EXAMPLES

The following examples are intended to further clarify the present invention, and are nowhere intended to limit the scope of the present invention.

### EXAMPLE 1

Step 1 A mixture of dimethyl 1,3-acetonedicarboxylate (1 equiv, 87.2 mmol, 15.2 g) and n-pentanol (3 equiv, 261.8 mmol, 23.08 g) was stirred at 140 °C, while MeOH was continuously removed under reduced pressure (800 mbar). When full conversion was achieved, the excess of n-pentanol was removed under reduced pressure and dipentyl 1,3-acetonedicarboxylate was obtained as an oil in 80% yield.

Step 2 A solution of 2-pyridine carboxaldehyde (2 equiv, 64.0 mmol, 6.86 g) in 2-methyl-1-propanol (40 mL) was added to a mixture of dipentyl 1,3-acetonedicarboxylate (1 equiv, 32.0 mmol, 9.17 g) in 2-methyl-1-propanol (60 mL). Methylamine (40 wt.% in H₂O, 1 equiv, 32.0 mmol, 2.49 g) was added drop-wise while the temperature was maintained below 20 °C. The reaction mixture was heated to 45 °C for 1 hour while H₂O was continuously removed under reduced pressure. To this crude mixture 2-picolylamine (1.1 equiv, 35.2 mmol, 3.81 g) was added after which formaldehyde (37 wt.% in H₂O, 2.2 equiv, 70.4 mmol, 5.72 g) was added drop-wise added over a period of 30 minutes. The mixture was stirred for 1.5 hours at 60 °C. Subsequently, the temperature was maintained at 65 °C while H₂O was continuously removed under reduced pressure (50 mbar). Half of the amount of crude material was purified by column chromatography (C18, H₂O/MeCN = 1/1 -> 0/1). The most pure fractions were combined and the solvent was removed by lyophilization to afford dipentyl 3-methyl-9-oxo-2,4-bis(2-pyridyl)-7-[(2-pyridyl)methyl]-3.7-diazabicyclo[3.3.1]nonane-1,5-dicarboxylate (2.88 g, 4.6 mmol, ligand 1A) as a mixture of product and corresponding hydrate, as a highly viscous substance with a purity of 98.4% (UPLC-UV, 254 nm) and 28% yield.

### EXAMPLE 2

Step 1 Dimethyl 1,3-acetonedicarboxylate (1 equiv, 43.1 mmol, 7.50 g) and 1-methoxy-2-propanol (3 equiv, 128 mmol, 11.50 g) were stirred at 140 °C while the formed methanol was continuously distilled of under vacuum (800 mbar) and extra 1-methoxy-2-propanol was added to compensate for its removal. The reaction mixture was stirred at 140 °C for 105 minutes, after which it was cooled to room temperature overnight. Subsequently, the stirring was continued at 140 °C for an additional 7.5 hours, until 98% conversion was obtained. Excess 1-methoxy-2-propanol was removed under reduced pressure (60 °C, 35 mbar). The resulting yellow oil was dried under high vacuum to afford 5.2 g di-2-methoxy-1-methylethyl 1,3-acetonedicarboxylate as a yellow oil (40% yield).

Step 2 Di-2-methoxy-1-methylethyl 1,3-acetonedicarboxylate (1 equiv, 17.05 mmol, 4.95 g) was stirred in isobutanol (33 mL) at room temperature in a water bath. A solution of 2-pyridinecarboxaldehyde (2 equiv, 34.10 mmol, 3.65 g) in isobutanol (22 mL) was added to the mixture dropwise. Subsequently, methylamine (40wt.% in H₂O, 1 equiv, 17.05 mmol, 1.32 g) was added to the mixture dropwise while keeping the internal temperature below 20 °C. The mixture was heated to 45 °C and water was removed as an azeotrope with isobutanol under reduced pressure. After 50 minutes, the mixture was cooled to room temperature and used as crude mixture for the following reaction (purity 95.6% UPLC-UV (254 nm)). 2-Picolylamine (1.1 equiv, 18.8 mmol, 1.93 mL) was added to this crude mixture (slurry in isobutanol, 1 equiv, 17.06 mmol) at room temperature and the mixture was placed in a water bath. Subsequently, formaldehyde (37 wt.% in H₂O, 2.2 equiv, 37.5 mmol, 2.79 mL) was added dropwise over 30 minutes. The temperature was adjusted to 60 °C and water was distilled off as an azeotrope with isobutanol under reduced pressure (800 mbar) during 105 minutes. The mixture was cooled to room temperature and stored in the freezer overnight, after which the reaction was continued at 60 °C under continuous distillation for an additional 120 minutes. Extra reagents were added at room temperature: 2-picolylamine (0.4 equiv, 6.82 mmol, 0.7 mL) and formaldehyde (37 wt.% in H₂O, 0.8 equiv, 13.6 mmol, 1 mL) and the reaction mixture was stirred at 60 °C under atmospheric pressure for 75 minutes. The solvent was removed under reduced pressure at 45 °C and the residue was purified by reversed phase column chromatography (C18, CH₃CN/H₂O = 1/9 -> 1/0). All fractions containing the product in sufficient purity were combined and dried to afford 3.1 g of di-2-methoxy-1-methylethyl 3-methyl-9-oxo-2,4-bis(2-pyridyl)-7-[(2-pyridyl)methyl]-3.7-diazabicyclo[3.3.1]nonane-1,5-dicarboxylate, as a mixture of isomers and hydrate (yield 28%, purity >99% UPLC-UV (254 nm), ligand 1B).

### EXAMPLE 3

Step 1 2-Pyridinecarboxaldehyde (4 equiv, 0.38 mol, 40.59 g) was dissolved in methanol (100 mL) and hexamethylenediamine (1 equiv, 0.09 mol, 11.01 g) was added. Dimethyl-1,3-acetonedicarboxylate (2 equiv, 0.19 mol, 33.0 g) was added to the mixture and the reaction mixture was stirred at 65 °C for an hour. The mixture was cooled to -20 °C resulting in the precipitation of material. The solids were isolated by filtration and washed with cold EtOH. The isolated material was dried under reduced pressure. Tetramethyl 1,1'-(hexane-1,6-diyl)bis(4-oxo-2,6-di(pyridin-2-yl)piperidine-3,5-dicarboxylate (33.7 g) was obtained with a purity of 90% (UPLC-UV, 254 nm) and 43% yield.

Step 2 Formaldehyde (37 wt.% in H₂O, 4.4 equiv, 180.6 mmol, 14.66 g) was slowly added to a solution of 2-picolylamine (2.2 equiv, 90.3 mmol, 9.77 g) in isobutanol (250 mL). Tetramethyl 1,1'-(hexane-1,6-diyl)bis(4-oxo-2,6-di(pyridin-2-yl)piperidine-3,5-dicarboxylate (1 equiv, 41.1 mmol, 33.7 g, 90% purity) was added and the mixture was stirred for 2 days at 50 °C. The reaction mixture was concentrated under reduced pressure and the crude product was purified by column chromatography (C18, H₂O/MeCN = 65/35 -> 0/1). The product fractions were partly concentrated to remove acetonitrile and then extracted with CH₂Cl₂ (3 x 500 mL). The combined organic layers were concentrated under reduced pressure. The material was purified by column chromatography (C18, H₂O/MeCN (65/35 -> 4/6). The product fractions were partly concentrated to remove acetonitrile, extracted with CH₂Cl₂ (3 x 150 mL), dried over MgSO₄ and concentrated under reduced pressure. Dimethyl 3-(6-{1,5-dimethoxycarbonyl-9-oxo-2,4-bis(2-pyridyl)-7-[(2-pyridyl)methyl]-3.7-diazabicyclo[3.3.1]non-3-yl}hexyl)-9-oxo-2,4-bis(2-pyridyl)-7-[(2-pyridyl)methyl]-3.7-diazabicyclo[3.3.1]nonane-1,5-dicarboxylate (mixture of isomers and corresponding hydrate) was obtained as a yellowish powder with a purity of 99.6% (UPLC-UV, 254 nm, yield 10%, ligand bis-3).

### COMPARATIVE EXAMPLE 4

Dimethyl 3-methyl-9-oxo-2,4-bis(2-pyridyl)-7-[(2-pyridyl)methyl]-3.7-diazabicyclo[3.3.1]nonane-1,5-dicarboxylate (BOC) is prepared according to procedures described in US 2014/114073 A1.

### COMPARATIVE EXAMPLE 5

Dimethyl 3-methyl-9-oxo-2,4-di(thiazol-2-yl)-7-(pyridin-2-ylmethyl)-3,7-diazabicyclo[3.3.1]nonane-1,5-dicarboxylate (2-TBP) is prepared according to procedures described in WO 2020/008205.

### EXAMPLES 6-8

Fe-complex of ligand 1A, Fe-complex of ligand 1B and Fe-complex of ligand bis-3 can be prepared according to procedures described in US 2014/114073 A1.

### EXAMPLE 9

A bispidine ligand (1A, 1B, 3-bis, BOC and 2-TBP) is mixed with iron (II) chloride tetrahydrate in ethanol until an homogeneous solution is obtained. Equimolar amounts of ligand and iron are used and the concentration of Fe in the solution is 0.05 wt.%. Next, this solution is added to a white alkyd paint formulation in an amount of 0.001 wt.% Fe. The used alkyd paint formulation is described in Table 1 and Table 2. The paint formulation is stored on shelf for a predetermined number of days - referred to as shelf life - at room temperature under an inert atmosphere, and is subsequently applied on a surface at a constant layer thickness of about 75 µm on glass plates and allowed to dry. An Elcometer ^{®} 5300 Ball Type Drying Time Recorder was used to determine the drying time of the white paints in a controlled climate of 20°C and 70% relative humidity. The time to dry-hard state of the composition is determined according to method ASTM D5895-03. The results are depicted in Figure 1.

| Table 1. Base formulation | content / wt.% |
|---|---|
| Valires, long oil alkyd (70% solids) | 56.22% |
| D40 aliphatic solvent | 14.63% |
| Lecithine emulsifier | 0.41% |
| Bentone rheology modifier | 0.10% |
| Kronos white pigment | 28.63% |

| Table 2. Formulation with driers and anti-skin agent | content / wt.% |
|---|---|
| Base formulation | 97.30% |
| Ca-neodecanoate solution (5% Ca) | 1.53% |
| D60 Zr-neodecanoate solution (18% Zr) | 0.21% |
| Ligand/Fe mix (0.05% Fe) | 0.77% |
| Anti-skinning agent | 0.20% |

Figure 1 shows that alkyd resin compositions comprising a mixture of iron ions and bispidine ligands BOC and 2-TBP exhibit a progressive loss of drying properties as evidenced by the enhanced time to dry-hard with longer shelf life. In contrast, alkyd resin compositions comprising a mixture of iron ions and bispidine ligands 1A, 1B and bis-3 do not suffer any significant performance loss up to a shelf life of about 3 months.

### EXAMPLE 10

A bispidine ligand (1A, 1B, 3-bis and BOC) is mixed with iron (II) chloride tetrahydrate in ethanol until an homogeneous solution is obtained. Equimolar amounts of ligand and iron are used and the concentration of Fe in the solution is 0.05 wt.%. Next, 1g of this solution is homogeneously added to 100 g of a low and medium reactive orthophthalic UPR resin. Then 1g of peroxide curing agent is added and the mixture is vigorously stirred for 30 seconds, after which the gelling is monitored with a Brookfield Model DV-III Ultra Rheometer equipped with a SC4-27 spindle. Gel time (minutes), peak exotherm time (minutes) and peak exotherm temperature (°C) are measured (Table 3 and 4).

**Table 3. UPR activity low reactive UPR resin.**

| | t _{gel} (min) | T ₘₐₓ (°C) | t to T ₘₐₓ (min) |
|---|---|---|---|
| 1A (0.05% Fe) | 8 | 105 | 25 |
| 1B (0.05% Fe) | 8 | 102 | 31 |
| 3-bis (0.05% Fe) | 8 | 103 | 24 |
| BOC (0.05% Fe) | 7 | 112 | 29 |

**Table 4. UPR activity medium reactive UPR resin.**

| | t _{gel} (min) | T ₘₐₓ (°C) | t to T ₘₐₓ (min) |
|---|---|---|---|
| 1A (0.05% Fe) | 5 | 139 | 12 |
| 1B (0.05% Fe) | 6 | 156 | 12 |
| 3-bis (0.05% Fe) | 3 | 166 | 9 |
| BOC (0.05% Fe) | 4 | 160 | 9 |

### EXAMPLE 11

The same white paint formulation was used out of Example 9 (Table 1 and 2) to evaluate the hardness development over time using Fe-bispidones solution 1A, 1B, 3-bis and BOC (0.05% Fe in ethanol). Films of 90 micron were applied on glass plates and the hardness (Persoz, seconds) was measured after 1, 7, 14 and 28 days (Table 5). A Persoz & König Pendulum Hardness Tester was used to determine the hardness of the films in a controlled climate of 20°C and 70% relative humidity.

**Table 5. Persoz hardness white paint (seconds).**

| | 1 day | 7 days | 14 days | 28 days |
|---|---|---|---|---|
| 1A (0.05% Fe) | 44 | 79 | 80 | 112 |
| 1B (0.05% Fe) | 48 | 81 | 82 | 108 |
| 3-bis (0.05% Fe) | 45 | 76 | 77 | 102 |
| BOC (0.05% Fe) | 52 | 87 | 89 | 118 |

### EXAMPLE 12

The same paint formulation was used out of Example 9 (Table 1 and 2) to evaluate the yellowing over time using Fe-bispidone solutions 1A, 1B, 3-bis and BOC (0.05% Fe in ethanol). Films of 90 micron were applied on glass plates and the yellowing was measured after 1, 28, 60 and 120 days (Table 6 and 7, light and dark respectively). A Minolta ^{®} Chroma meter CR-200 was used to determine the b*-value of the CIELAB colour space.

**Table 6. Yellowing (b*-values) in light.**

| | 1 day | 28 days | 60 days | 120 days |
|---|---|---|---|---|
| 1A (0.05% Fe) | -1,58 | -1,44 | -1,22 | -1,09 |
| 1B (0.05% Fe) | -1,01 | -1,03 | -0,78 | -0,63 |
| 3-bis (0.05% Fe) | -1,16 | -1,03 | -0,80 | -0,82 |
| BOC (0.05% Fe) | -1,24 | -1,29 | -1,09 | -0,93 |

**Table 7. Yellowing (b*-values) in dark.**

| | 1 day | 28 days | 60 days | 120 days |
|---|---|---|---|---|
| 1A (0.05% Fe) | -1,58 | -1,29 | -1,03 | -0,36 |
| 1B (0.05% Fe) | -1,01 | -0,86 | -0,55 | 0,19 |
| 3-bis (0.05% Fe) | -1,16 | -0,90 | -0,74 | -0,38 |
| BOC (0.05% Fe) | -1,24 | -0,95 | -0,74 | -0,28 |

### EXAMPLE 13

2 equiv. pyridine-2-aldehyde were added to a solution comprising 1 equiv. 1,3-acetonedicarboxylic acid dimethyl ester in EtOH at 0°C , and stirred for 15 minutes. Subsequently, a solution of methylamine (1 equiv., 40% solution in water) in EtOH was added dropwise, and the mixture was allowed to warm to room temperature, and then stirred for 30 min at 40°C. Cooling to room temperature affords a beige precipitate. 1.2 equiv. 2-aminomethyl-pyridine and a solution of 2.5 equiv. formaldehyde (37% in water) dissolved in EtOH were added to the resulting suspension. The mixture was heated for 1.5 h at 55 °C. After cooling to room temperature, white crystals were formed, which were collected by filtration, washed with cold EtOH, and dried under vacuum to yield the dimethyl bispidone ligand. The reaction is schematically shown below:

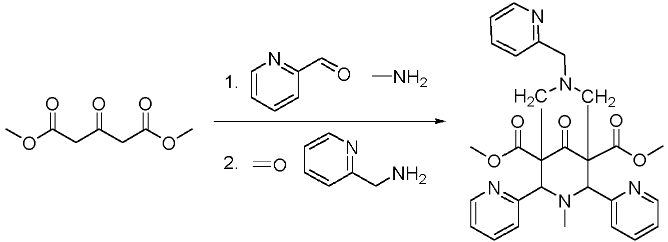

Subsequently, the aforementioned dimethyl bispidone is reacted with 1-methoxy-2-propanol, while to formed methanol is distilled off. The desired 1-methyl-2-methoxyethyl bispidone ligand is obtained in good yield. The reaction is schematically shown below:

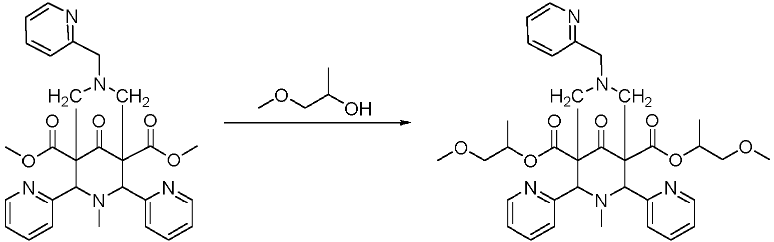

### EXAMPLE 14

The procedure according to Example 13 is repeated, whereby the obtained dimethyl bispidone is reacted with 1-pentanol, while to formed methanol is distilled off. The desired pentyl bispidone ligand is obtained in good yield.

### EXAMPLE 15

Di-2-methoxyethyl-1,3-acetonedicarboxylate (1 equiv, 335 mmol, 87.86 g) was stirred in isobutanol (167 mL) at 10°C. Next, a solution of 2-pyridinecarboxaldehyde (2 equiv, 670 mmol, 63 g) in isobutanol (108 mL) was added to the mixture dropwise over a period of 1 hour. Subsequently, methylamine (40 wt.% in H2O, 1 equiv, 335 mmol, 29 mL) was added to the mixture dropwise while keeping the internal temperature below 20 °C. The mixture was heated to 45 °C and water was removed as an azeotrope with isobutanol under reduced pressure. After 1 hour and 45 minutes, the mixture was cooled to room temperature and used as crude mixture for the following reaction. 2-Picolylamine (1.25 equiv, 420 mmol, 43 mL) was added to this crude mixture at room temperature together with isobutanol (47 mL). Subsequently, formaldehyde (37 wt.% in H₂O, 2.67 equiv, 890 mmol, 66 mL) was added dropwise over 110 minutes. The temperature was adjusted to 60°C and stirring continued for 2 hours. After 2 hours, the mixture was collected in a 1 L flask and concentrated using vacuum pumps (228.45 g of crude product). 1.08 g of this crude ligand was dissolved in acetonitrile (0.1 mL/g) and warmed to 50°C. 5 mL/g of water was added and the mixture vortexed until a precipitate was observed. The mixture was cooled overnight in the fridge, filtered and dried on the lyophylizer to yield 0.38 g of di-2-methoxyethyl 3-methyl-9-oxo-2,4-bis(2-pyridyl)-7-[(2-pyridyl)methyl]-3.7-diazabicyclo[3.3.1]nonane-1,5-dicarboxylate, as a mixture of product and corresponding hydrate (yield 35%, purity >98% UPLC-UV (254 nm), ligand 1C).

### EXAMPLE 16

Didecyl 1,3-acetonedicarboxylate (1 equiv, 95 mmol, 40.53 g) was stirred in isobutanol (48 mL) in an ice bath. A solution of 2-pyridinecarboxaldehyde (2 equiv, 190 mmol, 18 mL) in isobutanol (30 mL) was added to the mixture dropwise. Subsequently, methylamine (40 wt.% in H₂O, 1 equiv, 100 mmol, 8.2 mL) was added to the mixture dropwise while keeping the internal temperature below 20 °C. The mixture was heated to 45°C and water was removed as an azeotrope with isobutanol under reduced pressure. After 30 minutes, the mixture was cooled to room temperature and used as crude mixture for the following reaction. 2-Picolylamine (1.25 equiv, 120 mmol, 12.3 mL) was added to this crude mixture at room temperature together with isobutanol (13 mL). Subsequently, formaldehyde (37 wt.% in H₂O, 2.67 equiv, 250 mmol, 19 mL) was added dropwise over 30 minutes. The temperature was adjusted to 60 °C and stirring continued for 2 hours. After 2 hours, the mixture was concentrated using vacuum pumps (70 g of crude product), and was further purified using column chromatography to obtain didecyl 3-methyl-9-oxo-2,4-bis(2-pyridyl)-7-[(2-pyridyl)methyl]-3.7-diazabicyclo[3.3.1]nonane-1,5-dicarboxylate, as a mixture of product and corresponding hydrate (ligand 1D).

### EXAMPLE 17

Fe-complexes of bis(methoxyethyl) ligand (ligand 1C) and bisdecyl ligand (ligand 1D) were prepared according to procedures described in US 2014/114073 A1.

### EXAMPLE 18

A bispidine ligand (1C, 1D and BOC) is mixed with iron (II) chloride tetrahydrate in 1,2-propyleneglycol until an homogeneous solution is obtained. Equimolar amounts of ligand and iron are used and the concentration of Fe in the solution is 0.05 wt.%. Next, this solution is added to a white alkyd paint formulation in an amount of 0.001 wt.% Fe. The used alkyd paint formulation is described in Table 1 and 2. The paint formulation is stored on shelf for a predetermined number of days - referred to as shelf life - at room temperature under an inert atmosphere, and is subsequently applied on a surface at a constant layer thickness of about 75 µm on glass plates and allowed to dry. An Elcometer ^{®} 5300 Ball Type Drying Time Recorder was used to determine the drying time of the white paints in a controlled climate of 20°C and 70% relative humidity. The time to dry-hard state of the composition is determined according to method ASTM D5895-03. The results are depicted in Figure 1.

### EXAMPLE 19

A bispidine ligand (1C, 1D and BOC) is mixed with iron (II) chloride tetrahydrate in 1,2-propyleneglycol until an homogeneous solution is obtained. Equimolar amounts of ligand and iron are used and the concentration of Fe in the solution is 0.05 wt.%. Next, 1g of this solution is homogeneously added to 100 g of a low reactive orthophthalic UPR resin. Then 1 g of peroxide curing agent is added and the mixture is vigorously stirred for 30 seconds, after which the gelling is monitored with a Brookfield Model DV-III Ultra Rheometer equipped with a SC4-27 spindle. Gel time (minutes), peak exotherm time (minutes) and peak exotherm temperature (°C) are measured (Table 8).

**Table 8. UPR activity low reactive UPR resin.**

| | t _{gel} (min) | T ₘₐₓ (°C) | t to T ₘₐₓ (min) |
|---|---|---|---|
| 1C (0.05% Fe) | 7 | 92 | 20 |
| 1D (0.05% Fe) | 11 | 91 | 28 |
| BOC (0.05% Fe) | 6 | 84 | 20 |

### EXAMPLE 20

The same white paint formulation was used out of Example 9 (Table 1 and 2) to evaluate the hardness development over time using, 1C, 1D & BOC (0.05% Fe in 1,2-propyleneglycol). Films of 90 micron were applied on glass plates and the hardness (Persoz, seconds) was measured after certain days (Table 9). A Persoz & König Pendulum Hardness Tester was used to determine the hardness of the films in a controlled climate of 20°C and 70% relative humidity.

**Table 9. Persoz hardness white paint (seconds).**

| | 2 days | 11 days | 23 days | 34 days |
|---|---|---|---|---|
| 1C (0.05% Fe) | 59 | 79 | 101 | 95 |
| BOC (0.05% Fe) | 61 | 79 | 99 | 95 |

| | 1 day | 7 days | 14 days | 28 days |
|---|---|---|---|---|
| 1D (0.05% Fe) | 54 | 88 | 92 | 90 |
| BOC (0.05% Fe) | 48 | 74 | 88 | 87 |

### EXAMPLE 21

The same paint formulation was used out of Example 9 (Table 1 and 2) to evaluate the yellowing over time using Fe-bispidone solutions 1C and BOC (0.05% Fe in 1,2-propyleneglycol). Films of 90 micron were applied on glass plates and the yellowing was measured after 2, 22 and 69 days (Table 10 and 11, light and dark respectively). A Minolta ^{®} Chroma meter CR-200 was used to determine the b*-value of the CIELAB colour space.

**Table 10. Yellowing (b*-values) in light.**

| | 2 days | 22 days | 69 days |
|---|---|---|---|
| 1C (0.05% Fe) | -1,06 | -0,90 | -0,71 |
| BOC (0.05% Fe) | -0,62 | -0,93 | -0,39 |

**Table 11. Yellowing (b*-values) in dark.**

| | 2 days | 22 days | 69 days |
|---|---|---|---|
| 1C (0.05% Fe) | -1,06 | -1,00 | -0,53 |
| BOC (0.05% Fe) | -0,62 | -0,68 | -0,30 |

## Claims

1. A multidentate ligand L_{B} according to formula (I), wherein: 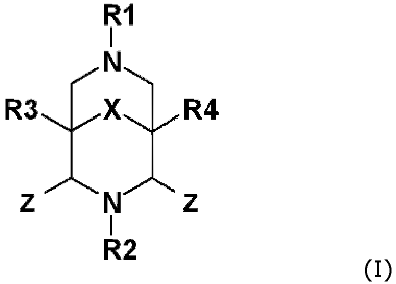
R1 and R2 are independently selected from the group consisting of:
- a group containing a heteroatom capable of coordinating to a transition metal;
- a -C1-C24-alkyl; and
- a -C1-C22-substituted-alkyl;
R3 and R4 are same and are selected from: -C(=O)O(C2-C4-alkyl-O-(C1-C4-alkyl)) and -(CH₂)n-C(=O)OR5, wherein n is 0 or 1, and wherein R5 is selected from the group consisting of: -C5-C12-alkyl;
X is -C(=O)-, a ketal of -C(=O)-, a hemiketal of -C(=O)-, or -C(R6)₂- wherein each R6 is independently selected from hydroxyl and -O-C1-C4-alkyl; and
z groups are 2-pyridinyl.

2. A multidentate ligand L_{B} according to claim 1, according to formula (I) wherein R1 is a group containing a heteroatom capable of coordinating to a transition metal, R2 is a -C1-C24-alkyl, R3 and R4 are same and are selected from: - C(=O)O(C2-C4-alkyl-O-(C1-C4-alkyl)) and -(CH₂)n-C(=O)OR5, wherein n is 0 or 1, and wherein R5 is selected from the group consisting of: -C5-C12-alkyl; X is -C(=O)-, a ketal of -C(=O)-, a hemiketal of -C(=O)-, or -C(R6)₂- wherein each R6 is independently selected from hydroxyl and -O-C1-C4-alkyl; and z groups are 2-pyridinyl.

3. A multidentate ligand L_{B} according to claim 1, according to formula (I) wherein R1 is pyridin-2-ylmethyl, R2 is methyl, R3 and R4 are same and selected from -C(=O)-O-C₅H₁₁, -C(=O)-O-C₆H₁₃, -C(=O)-O-C₇H₁₅ and -C(=O)-O-C₈H₁₇, X is -C(=O)- and z is 2-pyridinyl.

4. A multidentate ligand L_{B} according to claim 1, according to formula (I) wherein R1 is pyridin-2-ylmethyl, R2 is methyl, R3 and R4 are same and selected of - C(=O)O(C₂H₄-O-CH₃), -C(=O)-O-(1-methyl-2-methoxy-ethyl), X is -C(=O)- and z is 2-pyridinyl.

5. A transition metal complex comprising a transition metal and a multidentate ligand L_{B} according to any of claims 1 to 4.

6. A transition metal complex according to claim 5, wherein said transition metal is selected from Fe(II) and Fe(III).

7. A siccative composition comprising a mixture of a transition metal and a multidentate ligand L_{B} according to any of claims 1 to 4.

8. A siccative composition according to claim 7, wherein said transition metal is comprised in an amount of 0.001 to 1.00 wt.%, relative to the total weight of said siccative composition.

9. A siccative composition comprising a transition metal complex according to claim 5 or 6.

10. A siccative composition according to claim 9, wherein said transition metal is comprised in an amount of 0.001 to 1.00 wt.%, relative to the total weight of said siccative composition.

11. A curable liquid composition comprising: a) from 1 to 90 wt.% of an alkyd-based resin or of an unsaturated polyester resin; and, b) from 0.0001 to 1.0 wt.% of a siccative or curing catalyst, respectively, said siccative or curing catalyst comprising a mixture of a transition metal and a multidentate ligand L_{B} according to any of claims 1 to 4.

12. A curable liquid composition comprising: a) from 1 to 90 wt.% of an alkyd-based resin or of an unsaturated polyester resin; and, b) from 0.0001 to 1.0 wt.% of a siccative or curing catalyst, said siccative or curing catalyst comprising a transition metal complex according to claim 5 or 6.

13. A curable liquid composition according to claim 11, wherein said transition metal is selected from Fe(II) and Fe(III).

14. A curable liquid composition according to claim 12, wherein said transition metal is selected from Fe(II) and Fe(III).

15. Use of a transition metal complex according to claim 5 as a drying agent in a coating formulation.

## Patentansprüche

1. Mehrzähniger Ligand L_{B} gemäß Formel (I), wobei: 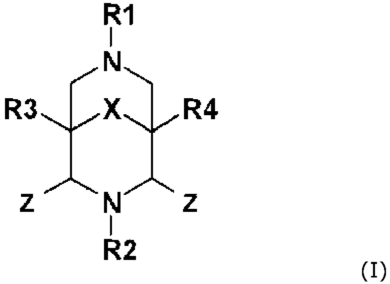
R1 und R2 unabhängig ausgewählt sind aus der Gruppe bestehend aus:
- einer Gruppe, die ein Heteroatom enthält, das an ein Übergangsmetall koordinieren kann;
- einem -Cl-C24-Alkyl; und
- einem -Cl-C22-substituierten Alkyl;
R3 und R4 sind gleich und ausgewählt aus: -C(=O)O(C2-C4-Alkyl-O-(Cl-C4-Alkyl)) und -(CH₂)n-C(=O)OR5, wobei n 0 oder 1 ist und wobei R5 ausgewählt ist aus der Gruppe bestehend aus: -C5-C12-Alkyl;
X ist -C(=O)-, ein Ketal von -C(=O)-, ein Hemiketal von - C(=O)- oder -C(R6)2-, wobei jedes R6 unabhängig ausgewählt ist aus Hydroxyl und -O-Cl-C4-Alkyl; und die z-Gruppen 2-Pyridinyl sind.

2. Mehrzähniger Ligand L_{B} nach Anspruch 1, gemäß Formel (I), wobei R1 eine Gruppe ist, die ein Heteroatom enthält, das an ein Übergangsmetall koordinieren kann, R2 ein -Cl-C24-Alkyl ist, R3 und R4 gleich sind und ausgewählt sind aus: -C(=O)O(C2-C4-Alkyl-O-(Cl-C4-Alkyl)) und -(CH₂)n-C(=O)OR5, wobei n 0 oder 1 ist und wobei R5 aus der Gruppe ausgewählt ist bestehend aus: -C5-C12-alkyl; X ist -C(=O)-, ein Ketal von -C(=O)-, ein Hemiketal von - C(=O)- oder -C(R6)2-, wobei jedes R6 unabhängig aus Hydroxyl und -O-Cl-C4-Alkyl ausgewählt ist; und die z-Gruppen 2-Pyridinyl sind.

3. Mehrzähniger Ligand L_{B} nach Anspruch 1, gemäß Formel (I), wobei R1 Pyridin-2-ylmethyl ist, R2 Methyl ist, R3 und R4 gleich sind und ausgewählt sind aus -C(=O)-O-C₅H₁₁, -C(=O)-O-C₆H₁₃, -C(=O)-O-C₇H₁₅ und -C(=O)-O-C₈H₁₇, X -C(=O)- ist und z 2-Pyridinyl ist.

4. Mehrzähniger Ligand L_{B} nach Anspruch 1 gemäß Formel (I), wobei R1 Pyridin-2-ylmethyl ist, R2 Methyl ist, R3 und R4 gleich sind und ausgewählt sind aus -C(=O)O(C₂H₄-O-CH₃), -C(=O)-O-(1-Methyl-2-methoxy-ethyl), X -C(=O)- ist und z 2-Pyridinyl ist.

5. Übergangsmetallkomplex, der ein Übergangsmetall und einen mehrzähnigen Liganden L_{B} nach einem der Ansprüche 1 bis 4 umfasst.

6. Übergangsmetallkomplex nach Anspruch 5, wobei das Übergangsmetall aus Fe(II) und Fe(III) ausgewählt ist.

7. Sikkative Zusammensetzung, die eine Mischung aus einem Übergangsmetall und einem mehrzähnigen Liganden L_{B} nach einem der Ansprüche 1 bis 4 umfasst.

8. Sikkative Zusammensetzung nach Anspruch 7, wobei das Übergangsmetall in einer Menge von 0,001 bis 1,00 Gew.-%, bezogen auf das Gesamtgewicht der sikkativen Zusammensetzung, enthalten ist.

9. Sikkative Zusammensetzung, die einen Übergangsmetallkomplex nach Anspruch 5 oder 6 umfasst.

10. Sikkative Zusammensetzung nach Anspruch 9, wobei das Übergangsmetall in einer Menge von 0,001 bis 1,00 Gew.-%, bezogen auf das Gesamtgewicht der sikkativen Zusammensetzung, enthalten ist.

11. Härtbare flüssige Zusammensetzung, umfassend: a) 1 bis 90 Gew.-% eines Harzes auf Alkydbasis oder eines ungesättigten Polyesterharzes; und b) 0,0001 bis 1,0 Gew.-% eines Sikkativ- oder Härtungskatalysators, wobei der Sikkativ- oder
Härtungskatalysator ein Gemisch aus einem Übergangsmetall und einem mehrzähnigen Liganden L_{B} nach einem der Ansprüche 1 bis 4 umfasst.

12. Härtbare flüssige Zusammensetzung, umfassend: a) 1 bis 90 Gew.-% eines Harzes auf Alkydbasis oder eines ungesättigten Polyesterharzes; und b) 0,0001 bis 1,0 Gew.-% eines Sikkativ- oder Härtungskatalysators, wobei der Sikkativ- oder Härtungskatalysator einen Übergangsmetallkomplex nach Anspruch 5 oder 6 umfasst.

13. Härtbare flüssige Zusammensetzung nach Anspruch 11, wobei das Übergangsmetall aus Fe(II) und Fe(III) ausgewählt ist.

14. Härtbare flüssige Zusammensetzung nach Anspruch 12, wobei das Übergangsmetall aus Fe(II) und Fe(III) ausgewählt ist.

15. Verwendung eines Übergangsmetallkomplexes nach Anspruch 5 als Trocknungsmittel in einer Beschichtungsformulierung.

## Revendications

1. Ligand polydenté L_{B} selon la formule (I), dans lequel : 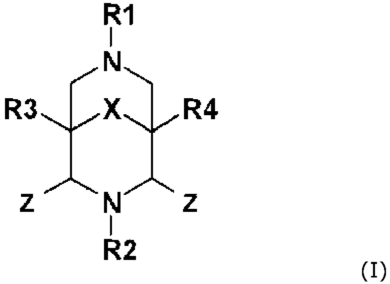
R1 et R2 sont indépendamment choisis dans le groupe constitué par :
- un groupe contenant un hétéroatome capable de se coordonner à un métal de transition ;
- un alkyl en C1-C24 ; et
- un alkyl substitué en C1-C22 ;
R3 et R4 sont identiques et sont choisis parmi : -C(=O)O(alkyl en C2-C4-O-(alkyl en C1-C4)) et -(CH₂)n-C(=O)OR5, dans lequel n est 0 ou 1, et dans lequel R5 est choisi dans le groupe constitué par : alkyl en C5-C12 ;
X est -C(=O)-, un cétal de -C(=O)-, un hémicétal de -C(=O)-, ou -C(R6)2-, dans lequel chaque R6 est indépendamment choisi parmi hydroxyle et -O-alkyl en C1-C4 ; et les groupes z sont 2-pyridinyle.

2. Ligand polydenté L_{B} selon la revendication 1, répondant à la formule (I) dans lequel R1 est un groupe contenant un hétéroatome capable de se coordonner à un métal de transition, R2 est un alkyl en C1-C24, R3 et R4 sont identiques et sont choisis parmi : - C(=O)O(alkyl en C2-C4-O-(alkyl en C1-C4)) et -(CH₂)n-C(=O)OR5, dans lequel n est 0 ou 1, et dans lequel R5 est choisi dans le groupe constitué par : alkyl en C5-C12 ; X est -C(=O)-, un cétal de -C(=O)-, un hémicétal de -C(=O)-, ou -C(R6)2-, dans lequel chaque R6 est indépendamment choisi parmi hydroxyle et -O-alkyl en C1-C4 ; et les groupes z sont 2-pyridinyle.

3. Ligand polydenté L_{B} selon la revendication 1, répondant à la formule (I) dans lequel R1 est pyridin-2-ylméthyle, R2 est méthyle, R3 et R4 sont identiques et choisis parmi -C(=O)-O-C₅H₁₁, -C(=O)-O-C₆H₁₃, -C(=O)-O-C₇H₁₅ et -C(=O)-O-C₈H₁₇, X est -C(=O)- et z est 2-pyridinyle.

4. Ligand polydenté L_{B} selon la revendication 1, répondant à la formule (I) dans lequel R1 est pyridin-2-ylméthyle, R2 est méthyle, R3 et R4 sont identiques et choisis parmi C(=O)O(C₂H₄-O-CH₃), - C(=O)-O-(1-méthyl-2-méthoxy-éthyl), X est -C(=O)- et z est 2-pyridinyle.

5. Complexe de métal de transition comprenant un métal de transition et un ligand polydenté L_{B} selon l'une quelconque des revendications 1 à 4.

6. Complexe de métal de transition selon la revendication 5, dans lequel ledit métal de transition est choisi parmi Fe(II) et Fe(III).

7. Composition siccative comprenant un mélange d'un métal de transition et d'un ligand polydenté L_{B} selon l'une quelconque des revendications 1 à 4.

8. Composition siccative selon la revendication 7, dans laquelle le métal de transition est présent en une quantité de 0,001 à 1,00 % en poids, par rapport au poids total de ladite composition siccative.

9. Composition siccative comprenant un complexe de métal de transition selon la revendication 5 ou 6.

10. Composition siccative selon la revendication 9, dans laquelle le métal de transition est présent en une quantité de 0,001 à 1,00 % en poids, par rapport au poids total de ladite composition siccative.

11. Composition liquide durcissable comprenant : a) de 1 à 90 % en poids d'une résine à base d'alkyde ou d'une résine de polyester insaturé ; et b) de 0,0001 à 1,0 % en poids d'un siccatif ou d'un catalyseur de durcissement, respectivement, ledit siccatif ou catalyseur de durcissement comprenant un mélange d'un métal de transition et d'un ligand polydenté L_{B} selon l'une quelconque des revendications 1 à 4.

12. Composition liquide durcissable comprenant : a) de 1 à 90 % en poids d'une résine à base d'alkyde ou d'une résine de polyester insaturé ; et b) de 0,0001 à 1,0 % en poids d'un siccatif ou d'un catalyseur de durcissement, ledit siccatif ou catalyseur de durcissement comprenant un complexe de métal de transition selon la revendication 5 ou 6.

13. Composition liquide durcissable selon la revendication 11, dans laquelle ledit métal de transition est choisi parmi Fe(II) et Fe(III).

14. Composition liquide durcissable selon la revendication 12, dans laquelle ledit métal de transition est choisi parmi Fe(II) et Fe(III).

15. Utilisation d'un complexe de métal de transition selon la revendication 5 en tant qu'agent de dessiccation dans une formulation de revêtement.
